# EUROPEAN PATENT SPECIFICATION

(11) **EP 3 068 872 B1**
(45) Date of publication and mention of the grant of the patent: **01.04.2020**
(21) Application number: 14861952.1
(22) Date of filing: 13.11.2014
(51) Int. Cl.: C12N 5/071

(54) **NON-EXPANDED POST-NATAL MULTILINEAGE-INDUCIBLE CELLS**
NICHT-EXPANDIERTE POSTNATALE MULTILINEAGE-INDUZIERBARE ZELLEN
CELLULES POST-NATALES NON AMPLIFIÉES INDUCTIBLES PAR MULTILIGNÉES

(30) Priority: 14.11.2013 US 201361904210 P
(43) Date of publication of application: 21.09.2016
(73) Proprietor: The University of Miami, Miami, Florida 33136 (US); The Government of the United States of America as Represented by the Department of Veterans Affairs, Washington DC 20420 (US)
(72) Inventor: SCHILLER, Paul C, Miami Beach, Florida 33140 (US); D'IPPOLITO, Gianluca, North Miami Beach, Florida 33179 (US)
(74) Representative: Trupiano, Federica
(86) International application number: PCT/US2014/065464
(87) International publication number: WO 2015/073677

(56) References cited:
- WO-A1-2011/050266
- WO-A1-2013/131192
- WO-A2-2004/069172
- WO-A2-2009/035612
- US-A1- 2005 221 476
- US-A1- 2008 095 748
- US-A1- 2011 189 211
- US-A1- 2011 300 112
- US-A1- 2012 021 482
- D'IPPOLITO, G ET AL.: 'Marrow-Isolated Adult Multilineage Inducible (MIAMI) Cells, A Unique Population Of Postnatal Young And Old Human Cells With Extensive Expansion And Differentiation Potential.' JOUMAL OF CELL SCIENCE vol. 117, no. 14, 14 January 2004, pages 2971 - 2981, XP002339338
- D ' Gianluca ET AL: "Non-Expanded MIAMI (ne-MIAMI) Cells Directly Isolated from Human Bone Marrow: Characterization, Interaction with Scaffolds and Therapeutic Applications", , 12 November 2013 (2013-11-12), XP055429057, Retrieved from the Internet: URL:https://ww2.eventrebels.com/ERImages/9 753/2392000/31748-2-11867.pdf [retrieved on 2017-11-27]
- Hyatt Regency ET AL: "The TERMIS-Americas 2013 Annual Conference & Exposition", , 10 November 2013 (2013-11-10), XP055429062, Retrieved from the Internet: URL:https://www.termis.org/docs/termisAM20 13_program.pdf [retrieved on 2017-11-27]
- Bruce Carter: "Miami VA part of international breakthrough in cell therapy", , 1 December 2013 (2013-12-01), pages 1-19, XP055429071, Retrieved from the Internet: URL:http://sfvafre.org/images/newsletter/R D_Newsletter_December_2013.pdf [retrieved on 2017-11-27]

## Description

### TECHNICAL FIELD

This invention relates to cells and methods of isolation in the absence of expansion and culturing to produce an unaltered source of post-natal multilineage-inducible cells for scientific and therapeutic uses.

### BACKGROUND OF THE INVENTION

In US Patent 7,807,458 B2 entitled "Multilineage-Inducible Cells and Uses Thereof', the inventors discovered a unique patented composition comprising isolated, post-natal, multilineage-inducible cells, which (i) express at least CD29, CD 81, CD90, CD122, and CD164; and (ii) do not express CD13 and another composition comprising isolated, post-natal, multilineage-inducible cells, which (i) express at least CD29, CD81, CD90, CD122, and CD164; and (ii) do not express CD34. This discovery was given the name MIAMI Cells.

The disclosed MIAMI cells have a unique molecular profile. For example, in one embodiment, MIAMI cells express at least one of CD29, CD81, CD90, or SSEA4 in combination with CD122, CD164, hepatocyte growth factor receptor (c-Met), bone morphogenetic protein receptor, type MB (BMP-receptor 1B), neurotrophic tyrosine kinase receptor type 3 (NTRK3), Oct-4, or Rex-1. MIAMI cells (and single-cell-derived colonies thereof) can be maintained in vitro without detectable changes in their characteristic molecular profile. Such in vitro MIAMI cell populations have multi-germ layer differentiation potential and can be differentiated into different mesodermal, neuroectodermal, and endodermal cell lineages.

MIAMI cells and differentiated MIAMI cells are useful for, among other things, the treatment of many types of diseases, including, for example, neurological disorders, bone disorders, cartilage disorders, and diabetes.

The work of Drs. Schiller and D'Ippolito recited in US 7,807,458 B2 is being incorporated by reference in the present invention by these same inventors who are providing unique and surprisingly unexpected improvement, and further refinement in the collection and harvesting of new post-natal, multipotent cells at exceptional yields and purities.

Historically, the collection of cells for transplantation has required culturing and expansion to provide sufficient quantities of viable cells. These procedures, while no doubt increasing the numbers of cells, can introduce contaminants from the culture medium and produce unknown alterations in the cells. Most cellular therapies require plating and ex-vivo expansion of cells before transplantation. These procedures can introduce chemicals to the cells that could alter their very structure. These cells are considered manipulated and as such require extensive testing and regulatory controls to insure both safety and efficacy.

The isolation and production procedures of culturing and expansion when conducted will likely result in some cellular manipulation, but the ability to achieve sufficient cell quantities has been heretofore unachievable absent this culturing and expansion. Furthermore, the culturing and expansion often leads to a more refined and directed cell expression which can be beneficial in its own right.

One attempt to achieve such a better procedure can be found in US 2011/0182866 A entitled "Isolation of Stem Cell Precursors and Expansion in Non-Adherent Conditions" by Ian K. McNiece of the University of Miami. In this publication, the inventor provides culture conditions for the generation of non-adherent stem cells by providing a closed culture system with a decreased probability of contamination and allow for passage without the use of enzyme treatment such as trypsin. He reported, mesenchymal stem cells (MSC) were grown in a closed bag system which enabled passage of the cells without the use of trypsin. The isolated non-adherent mesenchymal stem cells (NA-MSC) have a different phenotype to plastic adherent mesenchymal stem cells (PA-MSC) but maintained the ability to adhere to plastic. The non-adherent mesenchymal stem cells have a greater capacity to integrate into a tissue environment and continue to proliferate, resulting in regeneration of damaged tissue.

In this publication, a bag containing the cells was massaged to inhibit adherence, but wherein their culturing and expansion was still needed.

The present invention provides a unique composition of cells and new procedures overcoming these issues of cellular manipulation and does so in cell quantities sufficient for direct therapeutic use or as a source of high purity, multipotent cells for later culturing and expansion.

### SUMMARY OF THE INVENTION

A method of isolating non-expanded post-natal multilineage-inducible cells comprises the steps of: isolating bone marrow from the biological sample, isolating total nuclear cells (TNCs) obtained from the bone marrow, incubating the total nuclear cells in the presence of an antibody, separating the cells to isolate non-expanded post-natal multilineage-inducible cells and isolating the non-expanded post-natal multilineage-inducible cells in the absence of expansion, according to claim 1. The conditions for incubation (e.g., time, temperature, pH, and salt) may be predetermined for sufficient binding to the antibody or adhesion to the substrate. Preferably, the method when using the antibody uses anti SSEA-4 antibody phycoerythrin conjugated SSEA-4-PE. The antibody is directed to a cell surface marker selective for non-expanded post-natal multilineage-inducible cells (e.g., SSEA-4).

The step of separating the non-expanded post-natal multilineage-inducible cells includes separation by a magnetic or a cell sorting means. Alternatively, the step of separating the cells can include separation by differential cell separation using selective detachment solutions such as one or more of enzymes, citric acid, mild acids or divalent chelating agents or combinations thereof. The enzymes can be one of trypsin, collagenases or other mild proteases. The detachment solution when a mild acid can be citric acid, potassium chloride or combination thereof. The detachment solution when a chelating agent can be EDTA, EGTA or combination thereof.

The non-expanded post-natal multilineage-inducible cells collected yield 1.5% or greater of the total nuclear cells, typically 3.0 to 7.0% or nominal 4.0%, this represents over 3,500 times more cells than recovered under the original MIAMI cells. The final eluted product contains typically above 98%, in one sample 99.3 % of SSEA-4 cells, ne-MIAMI cells. Test samples of the non-expanded post-natal multilineage-inducible cells exhibit characteristics when expanded after being isolated by expressing phenotype markers SSEA-4, CD29, CD105, CD63, CD71 and CD164 post expansion. The non-expanded post-natal multilineage-inducible cells are multipotent cell precursors. The source animal is preferably a human donor. The invention further discloses a method of repairing and regenerating tissue in an animal (not according to the invention) having the steps of isolating cells from bone marrow of an animal, isolating non-expanded post-natal multilineage-inducible cells and transferring the non-expanded post-natal multilineage-inducible cells into the animal or another animal. The non-expanded post-natal multilineage-inducible cell recipient animal can also be the donor of the bone marrow. The non-expanded post-natal multilineage-inducible cells are obtained from allogeneic, autologous or syngeneic sources. The non-expanded post-natal multilineage-inducible cells are multipotent cells. The human donor can be living or a cadaver donor.

The predetermined temperature is above 0 degrees C to 10 degrees C, preferably about 4 degrees C. The predetermined time is 15 minutes to 60 minutes, preferably about 30 minutes. The cells, when later expanded, do not exhibit at least one of CD34, CD45, MHC-1 or HLA-DR phenotype markers, in a preferred embodiment none of these phenotype markers are exhibited. The invention, in one embodiment, is a composition having isolated non-expanded post-natal multilineage-inducible cells for direct transplantation into an animal. The cells further express at least one of hepatocyte growth factor receptor (c-Met), bone morphogenetic protein receptor type IB (BMP-receptor 1B), or neurotrophic tyrosine kinase receptor type (NTRK3). The cells are isolated from a biological sample comprising bone marrow and the cells are isolated from a mammal wherein the mammal is a human. The mammal can be a postmortem subject.

Also disclosed are compositions (not according to the invention) which can further have one or more natural or synthetic scaffolds. The scaffolds can be biodegradable scaffolds or non-biodegradable scaffolds or bioabsorbable scaffolds. The biodegradable scaffolds can be one of a gel, a water soluble monomer or a combination thereof. The non-biodegradable scaffolds can be one or more of bone, cartilage, tissue membrane or combinations thereof. The bioabsorbable scaffolds can be collagen sponge. The scaffolds can have releasable trophic factors that induce a cellular response, wherein the trophic factors include one or more of growth factor, vitamins, cytokines, morphogens, nucleotides, microRNA or other signaling molecules.

Also disclosed are pharmaceutical compositions (not according to the invention) having multilineage-inducible cells in a pharmaceutically acceptable carrier wherein the carrier is water, physiological saline, balanced salt solutions, aqueous dextrose, glycerol or the like.

### BRIEF DESCRIPTION OF THE DRAWINGS

The invention will be described by way of example and with reference to the accompanying drawings in which:

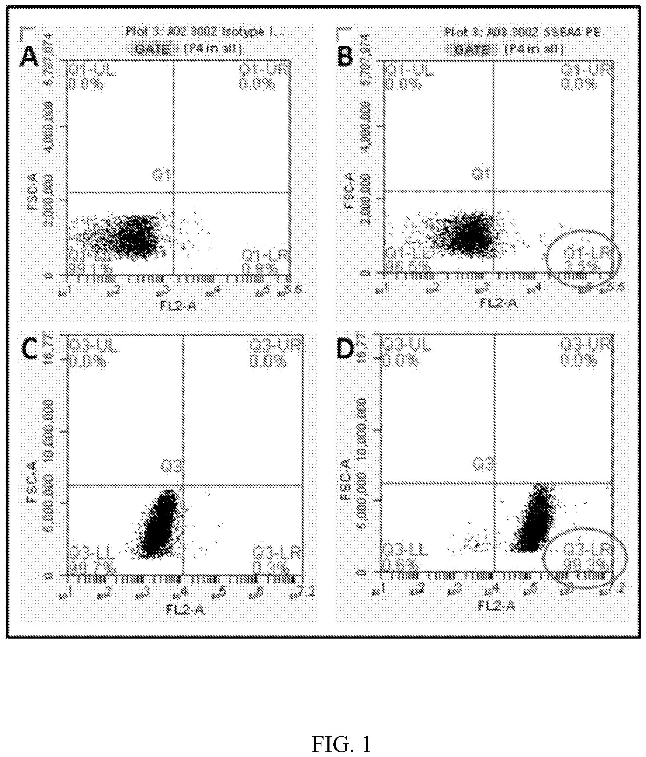

FIG. 1 is a set of graphs showing FACS analysis with anti SSEA-4. FIG. 1A is an MNCs analysis of Isotype-matched negative controls. FIG. 1B is an MNCs analysis with MNCs having 3.5% of SSEA-4 positive cells. FIG. 1C shows Isotype-matched negative controls after magnetic separation of SSEA-4 cells. FIG. 1D shows all the isolated cells are SSEA-4 positive after magnetic separation.

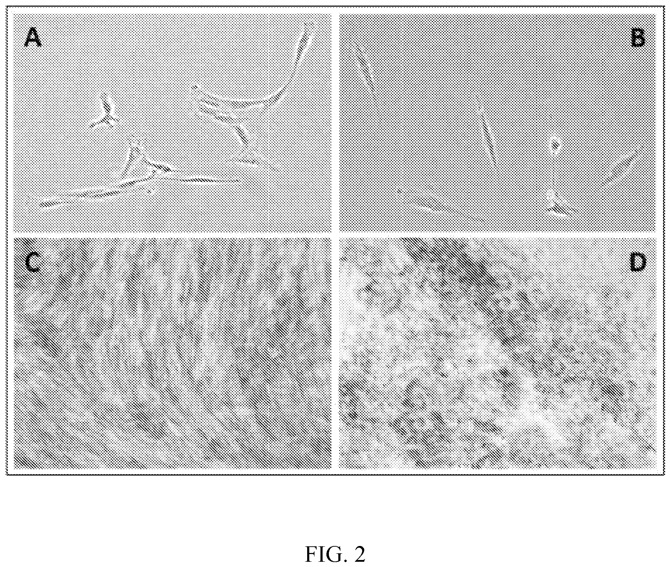

FIG. 2 is a set of photographs showing ne-MIAMI cells as they differentiate into bone forming cells. After isolation cells were expanded as adherent shown in figures 2A and 2B. Osteogenic differentiation medium is added to the culture, after 21 days in culture, ne-MIAMI cells were able to express osteoblastic markers: alkaline phosphatase shown in figure 2C and calcium deposition shown in figure 2D.

### ABBREVIATIONS

α-MEM: α-minimum essential medium
BDNF: brain-derived neurotrophic factors
Beta2/NeuroD: neurogenic differentiation transcription factor (also, beta-cell E-box transactivator 2)
bFGF: basic fibroblast growth factor
BHA: butylated hydroxyanisole
βME: β-mercaptoethanol
BMMNCs: bone marrow mononuclear cells
BMP-receptor 1B: bone morphogenetic protein receptor, type IB
BSP: bone sialoprotein
c-Met: HGF receptor
CNTFR: ciliary neurotrophic factor receptor
DMEM: Dulbecco's modified Eagle medium
DMSO: dimethyl sulfoxide
ECM: extracellular matrix
EF1-α: translation elongation factor 1-alpha
EGF: epidermal growth factor
ES: embryonic stem [cells]
FACS: fluorescence-activated cell sorting
FBS: fetal bovine serum
FN: fibronectin
GFAP: glial fibrillary acidic protein
GlyA: glycophorin-A
HGF: hepatocyte growth factor
ISL-1: islet-1 transcription factor
LPL: lipoprotein lipase
MAPCs: multipotent adult progenitor cells
MSCs: marrow stromal cells
NeuN: neuronal nuclear protein
NF160: neurofilament-160 kD
NF-L: neurofilament protein, light chain (68 kD)
NF-M 125 kD: neurofilament protein
NGF: β-nerve growth factor
Nkx6.1: NK6 transcription factor related, locus 1
NSCs: neural stem cells
NT-3: neurotrophin-3
NTRK3: NT-3 receptor
OC: osteocalcin
OP: osteopontin
PPAR-γ2: peroxisome proliferator activated receptor γ-2
Runx2: runt-homology domain transcription factor
SSEA4: stage-specific embryonic antigen 4
TGF-β3: transforming growth factor-β3
Trk-A tyrosine kinase receptor A (also, NGF receptor)
TuJ1: monoclonal antibody specific for β-III-tubulin

### TERMS

Unless otherwise noted, technical terms are used according to conventional usage. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

In order to facilitate review of the various embodiments of the invention, the following explanations of specific terms are provided:

Adherent: Connected to, associated with, or affixed to, a substrate. For example, a cell that adheres to a cell culture dish in vitro, and grows attached thereto is adherent. Typically, an adherent cell will not wash off a surface to which it is attached by gentle washing with a buffered saline solution. In some cases, enzymatic solutions (such as trypsin-EDTA) may be used to disrupt the attachment between an adherent cell and the surface to which it is attached. In other circumstances, adherent cells may be physically detached from a surface using a tool designed for such purposes, such as a cell scraper.

A non-adherent cell is one that is not stably connected to, associated with, or affixed to a substrate. Cells grown in suspension culture are examples of non-adherent cells.

β cells: Mature insulin producing cells. In vivo, these cells are found in the pancreatic islets of Langerhans. "β-like cells" are cells that express one or more markers characteristic of β cells, such as Nkx6.1 or insulin.

Biological Sample: Any sample that may be obtained directly or indirectly from a living or postmortem subject (such as, a recently deceased), including whole blood, plasma, serum, bone marrow, vertebral bodies, iliac crest aspirate, umbilical cord blood, tears, mucus, saliva, urine, pleural fluid, spinal fluid, gastric fluid, sweat, semen, vaginal secretion, sputum, fluid from ulcers and/or other surface eruptions, blisters, abscesses, and/or extracts of tissues, cells or organs (such as, fat, cartilage, muscle, skin, bone, teeth, liver, brain). The biological sample may also be a laboratory research sample such as a cell culture supernatant. The sample is collected or obtained using methods well known to those skilled in the art.

Bone morphogenetic protein receptor, type IB (BMP-receptor 1B or BMPR1B): A member of a family of transmembrane serine/threonine kinases, which are receptors for members of the TGF-β superfamily. Human BMPR1B cDNA encodes a 502-amino acid polypeptide that contains a single transmembrane domain and an intracellular serine/threonine kinase domain. BMPR1B mRNA is about 6.5 kb and is expressed in several human tissues, with highest levels in prostate and brain. BMPR1B is also known as activin receptor-like kinase 6 (or ALK6). (See, for example, ten Dijke et al., Science, 264:101-104, 1994; Ide et al., Oncogene, 14:1377-1382, 1997; Ide et al., Cytogenet. Cell Genet., 81:285-286, 1998).

CD29: A 130-kD antigen expressed, for example, in leukocytes. CD29 is also known as the β1-integrin subunit, which associates with CD49a in VLA-1 integrin. Alternate names for CD29 include Fibronectin Receptor, Beta Subunit (FNRβ), Very Late Activation Protein, Beta (VLA-β).

CD34: is a cluster of differentiation molecule present on certain cells within the human body. It is a cell surface glycoprotein and functions as a cell-cell adhesion factor. It may also mediate the attachment of stem cells to bone marrow extracellular matrix or directly to stromal cells. CD34 is also the name for the human gene that encodes the protein. The CD34 protein is a member of a family of single-pass transmembrane sialomucin proteins that show expression on early hematopoietic and vascular-associated tissue.

CD45: a 200-220kDa antigen, originally called leukocyte common antigen, which is encoded by the gene protein tyrosine phosphatase, receptor type, C also known as PTPRC. CD45, in its various isoforms, is present on all differentiated hematopoietic cells except erythrocytes and plasma cells, and assists in the activation of those cells.

CD49b is also known as the α2-integrin subunit. It associates with CD29 (the β1-integrin subunit) to bind collagen and laminin. Alternate names for CD49b are Very Late Activation Protein 2 Receptor, VLA-2 Receptor, or Platelet Glycoprotein Ia/IIa.

CD63: antigen is a protein that in humans is encoded by the *CD63* gene. This encoded protein is a cell surface glycoprotein that is known to complex with integrins. CD63 is mainly associated with membranes of intracellular vesicles, although it is also expressed in the cell surface.

CD71: An antigen ubiquitously distributed on the cell surface of actively growing human cells. It is a glycoprotein composed of disulfide-linked polypeptide chains, each of about 90 kD molecular weight. CD71 is also known as the transferrin receptor.

CD73: Is an antigen in the surface of many human cells. CD73 is an enzyme that in humans is encoded by the *NT5E* gene, the encode enzyme is 5'-nucleotidase (5'-NT), also known as ecto-5'-nucleotidase.

CD81: A 26-kD integral membrane protein, also known as TAPA1, which is expressed on many human cell types (including lymphocytes). CD81 is believed to associate with CD19 and CD21 to form B cell coreceptor. CD81 is a member of the transmembrane pore integral membrane protein family.

CD90: An 18-kD glycoprotein antigen expressed, for example, on CD34⁺ human prothymocytes, fibroblasts and brain cells and on mouse T cells. CD90 is also known as Thy-1. It belongs to immunoglobulin supergene family, and consists of a single immunoglobulin homology unit that is either intermediate between V and C or somewhat more similar to a V homology unit.

CD105: is a type I membrane glycoprotein located on cell surfaces and is part of the TGF beta receptor complex. It is also commonly referred to as endoglin, END, FLJ41744, HHT1, ORW and ORW1.

CD122: The 75-kD 6-chain of the interleukin-2 receptor (also known as, IL-2Rβ). This antigen is expressed, for example, in natural killer cells, resting T cells and, some B cell lines.

CD164: A protein antigen of about 80 to 90 kD, which is expressed, for example, in human CD34⁺ hematopoietic progenitor cells (Zannettino et al., Blood, 92:2613-2628, 1998). CD164 belongs to a heterogeneous group of secreted or membrane-associated proteins called sialomucins. Sialomucins are believed to have two opposing functions in vivo: first, as cytoprotective or antiadhesive agents and, second, as adhesion receptors.

Differentiation: A process whereby relatively unspecialized cells (for example, undifferentiated cells, such as multilineage-inducible cells) acquire specialized structural and/or functional features characteristic of mature cells. Similarly, "differentiate" refers to this process. Typically, during differentiation, cellular structure alters and tissue-specific proteins appear. "Osteogenic differentiation" is a process whereby undifferentiated cells acquire one or more properties (for example, morphological, biochemical, or functional properties) characteristic of bone-related cells, such as osteoblasts or osteoclasts. "Chondrogenic differentiation" is a process whereby undifferentiated cells acquire one or more properties (for example, morphological, biochemical, or functional properties) characteristic of cartilage-related cells, such as chondrocytes. "Adipogenic differentiation" is a process whereby undifferentiated cells acquire one or more properties (for example, morphological, biochemical, or functional properties) characteristics of adipocytes. "Neural differentiation" is a process whereby undifferentiated cells acquire one or more properties (for example, morphological, biochemical, or function properties) characteristic of neural cells, such as glial cells, oligodendrocytes, or neurons. "Neuronal differentiation" is a process whereby undifferentiated cells acquire one or more properties (for example, morphological, biochemical, or function properties) characteristic of neurons. "β-cell-like differentiation" is a process whereby undifferentiated cells acquire one or more properties (for example, morphological, biochemical, or functional properties) characteristic of a pancreatic β-cell.

Expand: A process by which the number or amount of cells in a cell culture is increased due to cell division. Similarly, the terms "expansion" or "expanded" refers to this process. The terms "proliferate," "proliferation" or "proliferated" may be used interchangeably with the words "expand," "expansion", or "expanded." Typically, during an expansion phase, the cells do not differentiate to form mature cells.

Expression: The process by which the coded information of a gene is converted into an operational, non-operational, or structural part of a cell, often including the synthesis of a protein.

Thus, the term "expression" contemplates either or both gene expression, measured for example, by levels of RNA (such as, mRNA) in a cell, or protein expression. Methods of determining RNA levels are well known in the art, and include Northern blots, RT-PCR, RNAse protection, and others.

Methods of determining protein expression are similarly well known, and include Western blots, functional assays, immunofluorescence, optical absorbance, microscopy (including electron microscopy) and others.

Extracellular matrix (ECM): A complex network of different combinations of collagens, proteoglycans (PG), hyaluronic acid, laminin, fibronectin, and many other glycoproteins. The ECM is a scaffold that fills extracellular spaces. In some instances, the ECM (or particular components thereof) can mediating cell-to-cell interactions, or play a functional role in mediating cellular proliferation or differentiation.

Proteoglycans may be modified by glycosaminoglycans (GAGs), which are long-chain compounds of repeated disaccharide units. The four main types of GAGs consist mainly of sulfated heparan sulfate/heparin, chondroitin sulfate/dermatan, keratan sulfate, and the non-sulfated glycosaminoglycan hyaluronic acid. Many proteoglycans contain a core protein which links them to the cellular membrane. Hyaluronic acid is the only extracellular oligosaccharide that is not known to be covalently linked to a protein.

Growth factor: A substance that promotes cell growth, survival, and/or differentiation. Growth factors include molecules that function as growth stimulators (mitogens), molecules that function as growth inhibitors (e.g. negative growth factors) factors that stimulate cell migration, factors that function as chemotactic agents or inhibit cell migration or invasion of tumor cells, factors that modulate differentiated functions of cells, factors involved in apoptosis, or factors that promote survival of cells without influencing growth and differentiation Examples of growth factors are bFGF, EGF, CNTF, HGF, NGF, and activin-A.

Hepatocyte growth factor receptor (c-Met): A tyrosine kinase comprised of disulfide-linked subunits of about 50 kD (alpha) and about 145 kD (beta), which is a receptor for hepatocyte growth factor. In the fully processed c-Met product, the alpha subunit is extracellular, and the beta subunit has extracellular, transmembrane, and tyrosine kinase domains as well as sites of tyrosine phosphorylation. (See, for example, Bottaro et al., Science, 251:802-804, 1991).

HLDR: is a major histocompatibility (MHC) class II cell surface receptor encoded by the human leukocyte antigen complex on chromosome 6 region 6p21.31. HLA (human leukocyte antigens) were originally defined as cell surface antigens that mediate graft-versus-host disease, which resulted in the rejection of tissue transplants in HLA-mismatched donors.

Induce: To cause to move forward to a result. For example, certain cell culture conditions may establish an environment that prompts one or more events (sometime, a cascade of events), which results in the specialization of a previously unspecialized cell type. In this example, placing an unspecialized cell into such culture conditions induces the cell to become more specialized, such as to differentiate.

Isolated: An "isolated" cell is a cell that has been purified from the other cellular components of a tissue. Cells can be isolated by a variety of methods, including mechanical and/or enzymatic methods. In one embodiment, an isolated population of cells includes greater than about 50%, greater than about 75%, greater than about 90%, greater than about 95%, or greater than about 99% of the cells of interest In another embodiment, an isolated population of cells is one in which no other cells of a different phenotype can be detected. In a further embodiment, an isolate population of cells is a population of cells that includes less than about 5%, or less than about 1% of cells of a different phenotype than the cells of interest. An "isolated" cell may be a population of clonally derived cells, such as cells expanded into a single-cell-derived colony.

Low density: A relatively small number of cells per unit area of a container in which the cells are contained. Adherent cells are often considered to be at low density when the population of cells do not form a continuous monolayer on the surface on which the cells are adhered. Exemplary low cell densities include no more than about 10⁴ cells/cm², or no more than about 10⁵ cells/cm².

Low-oxygen tension (or low oxygen conditions): Any culturing conditions below the normal atmospheric oxygen level (which is approximately 21%). Thus, in particular embodiments, low oxygen conditions are less than about 15% oxygen, less than about 10% oxygen, less than about 5% oxygen, or less than about 3% oxygen. In some embodiments, the culture oxygen conditions are kept as close as possible to the normal physiological oxygen conditions in which a particular cell would be found in vivo. This may mean that the oxygen conditions employed for a particular cell type will depend on the regional origin of that particular cell type. For example, cells from an alveolar origin may prefer growth at about 14% O2; cells from an arterial source will prefer an oxygen concentration of about 12%; whereas those from certain regions of the brain may prefer oxygen conditions as low as about 1.5%. Low oxygen conditions are not to be considered the same as "hypoxic" conditions. Low oxygen conditions are intended to mimic physiological conditions, whereas hypoxic conditions describe oxygen levels that are less than normal physiological conditions for a particular cell type.

Marker: A protein, glycoprotein, or other molecule expressed on the surface of a cell, which serves to help identify the cell. A cell surface marker can generally be detected by conventional methods. Specific, non-limiting examples of methods for detection of a cell surface marker are immunohistochemistry, fluorescence activated cell sorting (FACS), or an enzymatic analysis.

MHC-I: Are one of two primary classes of major histocompatibility complex (MHC) molecules (the other being MHC class II) and are found on nearly every nucleated cell of the body. Their function is to display fragments of proteins from within the cell to T cells; healthy cells will be ignored, while cells containing foreign proteins will be attacked by the immune system. Because MHC class I molecules present peptides derived from cytosolic proteins, the pathway of MHC class I presentation is often called the *cytosolic* or *endogenous pathway.*

Multilineage-inducible cell: A cell capable of differentiating into more than one cell lineage. A multilineage cell is capable of differentiating into cell types derived from more tam one germ layer, including cell types of mesodermal, ectodermal or endodermal origin. In particular examples, a multilineage-inducible cell can be differentiated into mesodermal, neuroectodermal, and endodermal cell lineages, including, for instance, osteoblasts, chondrocytes, adipocytes, neurons, and β-like cells.

Neurological disorder: A disorder in the nervous system, including the central nervous system or the peripheral nervous system. The term "neurological disorder" includes neurogenerative disorders. A "neurogenerative disorder" is an abnormality in the nervous system of a subject, such as a mammal, in which neural integrity is threatened. Without being bound by theory, neural integrity can be threatened when neural cells display decreased survival or when the neurons can no longer propagate a signal. Specific, non-limiting examples of neurological disorders are provided in the specification.

Neurotrophin-3 receptor (NTRK3) (also known as gp145 and trkC): A member of the TRK family of tyrosine protein kinase genes, which is expressed, for example, regions of the brain, including the hippocampus, cerebral cortex, and the granular cell layer of the cerebellum NTRK3 is a glycoprotein of about 145 kD, and a receptor for neurotrophin-3 (see, for example, Lamballe et al., Cell, 66:967-979, 1991; Valent et al., Europ. J. Hum. Genet., 5:102-104, 1997; McGregor et al., Genomics, 22:267-272, 1994).

Oct-4: A known developmentally regulated, mammalian transcription factor containing the POU homeo domain, which is characteristically expressed in undifferentiated pluripotent embryonic stem cells (see, for example, Flasza et al., Cloning Stem Cells, 5:339-354, 2003; Bhattacharya et al., Blood, 103:2956-2964, 2003; Sui et al., Differentiation, 71: 578-585, 2003; Palmieri et al., Dev. Biol., 166:259-267, 1994).

Pharmaceutically acceptable carriers: The pharmaceutically acceptable carriers useful in this invention are conventional. Remington's Pharmaceutical Sciences, by E. W. Martin, Mack Publishing Co., Easton, Pa., 15th Edition (1975), describes compositions and formulations suitable for pharmaceutical delivery of the multilineage-inducible cells herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

Post-natal: After birth. For example, a neonate (a newborn), a child, an adolescent, or an adult (including, for example, an aged adult).

Rex-1 (also known as Zfp-42): A known mammalian transcription factor containing zinc finger motifs, which is characteristically expressed in undifferentiated pluripotent embryonic stem cells (see, for example, Hosler et al., Mol. Cell. Biol., 9:5623-5629, 1989; Rogers et al., Development, 113:815-824, 1991; Hosler et al., Mol. Cell. Biol., 13:2919-2928, 1993; Nishiguchi et al., J. Biochem. (Tokyo), 116:128-139, 1994).

Stage-specific embryonic antigen 4 (SSEA4): A globoseries glycolipid (related to SSEA1 and SSEA3) recognized by monoclonal antibodies originally raised to distinguish early stages of mouse development Primate pluripotent cells express SSEA-4 and SSEA-3, while SSEA-1 is expressed only upon differentiation of such cells. (See, for example, Andrews et al., Int. J. Cancer, 66:806-816, 1996; Thomson and Marshall, Curr. Topics Dev. Biol., 38:133-165, 1998; Thomson et al., Science, 282:1145-1147, 1998).

Subject: Any living or postmortem mammal, such as humans, non-human primates, pigs, sheep, cows, rodents and the like which is to be the recipient of the particular treatment. In one embodiment, a subject is a human subject or a murine subject. Death of a subject is determined by any standard known in the art, including, for example, cessation of heart or brain function. A postmortem subject typically will have been dead for less than 48 hours, such as less than 24 hours. A postmortem subject may be housed in an environment that may slow cellular degradation, which occurs following death; for example, a cool environment (such as between about 0 degrees C. and about 15 degrees C., or between about 0 degrees C. and about 10 degrees C. between about 0 degrees C. and about 4 degrees C.) may slow cellular degradation.

Therapeutically effective amount of a cell: An amount of a MIAMI cell (or a differentiated MIAMI cell) that can be determined by various methods, including generating an empirical dose-response curve, potency and efficacy modeling, and other methods used in the biological sciences. In general, a therapeutically effective amount of MIAMI cells (or differentiated MIAMI cells) is an amount sufficient to alleviate at least one symptom of a disease or disorder to be treated in a subject. In one embodiment, a therapeutically effective amount of MIAMI cells (or differentiated MIAMI cells) is more than about 10,000 cells, more than about 20,000 cells, more than about 30,000 cells, or between about 5,000 cells and about 50,000 cells.

The therapeutically effective amount of cells will be dependent on the subject being treated (for example, the species or size of the subject), the degree that the subject is compromised, and the method and/or location of administration of the cells. In one embodiment, a therapeutically effective amount of cells is an amount of cells sufficient to measure MIAMI cells in the peripheral blood of a recipient.

Transduced and Transformed: A virus or vector "transduces" a cell when it transfers nucleic acid into the cell. A cell is "transformed" by a nucleic acid transduced into the cell when the DNA becomes stably replicated by the cell, either by incorporation of the nucleic acid into the cellular genome, or by episomal replication. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

Transplantation: The transfer of a tissue or an organ, or cells, from one body or part of the body to another body or part of the body. An "allogeneic transplantation" or a "heterologous transplantation" is transplantation from one individual to another, wherein the individuals have genes at one or more loci that are not identical in sequence in the two individuals. An allogeneic transplantation can occur between two individuals of the same species, who differ genetically, or between individuals of two different species. An "autologous transplantation" is a transplantation of a tissue or cells from one location to another in the same individual, or transplantation of a tissue or cells from one individual to another, wherein the two individuals are genetically identical.

Treating a disease: Refers to inhibiting (or preventing) the partial or full development or progression of a disease, for example in a person who is known to have a predisposition to a disease. An example of a person with a known predisposition is someone with a history of diabetes in the family, or who has been exposed to factors that predispose the subject to a condition, such as lupus or rheumatoid arthritis. Moreover, treating a disease refers to a therapeutic intervention that ameliorates at least one sign or symptom of a disease or pathological condition, or interferes with a pathophysiological process, after the disease or pathological condition has begun to develop.

Unless otherwise explained, all technical and scientific terms used herein have the same meaning as commonly understood by one of ordinary skill in the art to which this invention belongs. The singular terms "a," "an," and "the" include plural referents unless context clearly indicates otherwise. Similarly, the word "or" is intended to include "and" unless the context clearly indicates otherwise. "Comprising" means "including." Hence "comprising A or B" means including A or B, or including A and B. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of the present invention, suitable methods and materials are described below. In case of conflict, the present specification, including explanations of terms, will control. In addition, the materials, methods, and examples are illustrative only and not intended to be limiting.

Except as otherwise noted, the methods and techniques of the present invention are generally performed according to conventional methods well known in the art and as described in various general and more specific references that are cited and discussed throughout the present specification. See, e.g., Sambrook et al., Molecular Cloning: A Laboratory Manual, 2nd ed., Cold Spring Harbor Laboratory Press, 1989; Sambrook et al., Molecular Cloning: A Laboratory Manual, 3rd ed., Cold Spring Harbor Press, 2001; Ausubel et al., Current Protocols in Molecular Biology, Greene Publishing Associates, 1992 (and Supplements to 2000); Ausubel et al., Short Protocols in Molecular Biology: A Compendium of Methods from Current Protocols in Molecular Biology, 4th ed., Wiley & Sons, 1999; Harlow and Lane, Antibodies: A Laboratory Manual, Cold Spring Harbor Laboratory Press, 1990; and Harlow and Lane, Using Antibodies: A Laboratory Manual, Cold Spring Harbor Laboratory Press, 1999.

### DETAILED DESCRIPTION OF THE INVENTION

Original ad-MIAMI cells as disclosed herein are isolated post-natal, multilineage-inducible cells (MIAMI cells), which express a unique set of molecular markers. A summary of selected markers expressed (or not expressed, as applicable) in various MIAMI cell embodiments is shown in Table 1.

**TABLE 1**

| Representative MIAMI Cell Markers | |
|---|---|
| Marker | MIAMI Cell Expression |
| CD10 | + |
| CD13 | - |
| CD29 | + |
| CD34 | - |
| CD36 | - |
| CD44 | + |
| CD45 | - |
| CD49b | - |
| CD49e | + |
| CD54 (ICAM-1) | - |
| CD56 (NCAM) | - |
| CD63 | + |
| CD71 | - |
| CD81 (TAPA-1) | + |
| CD90 | + |
| CD103 | + |
| CD109 | - |
| CD117 (cKit) | - |
| CD122 (IL-2Rβ) | + |
| CD133 | - |
| CD156 | + |
| CD164 | + |
| BMP-receptor 1B | + |
| CNTFR | + |
| HGF Receptor (c-Met) | + |
| Class I-HLA | - |
| HLA-DR | - |
| hTERT | + |
| NTRK3 | + |
| POU4F1 (Oct-4) | + |
| Rex-1 | + |
| SSEA4 | + |

MIAMI cells may be identified by any unique set of the markers set forth in Table 1. For example, MIAMI cells may uniquely express at least one, at least two, at least three, at least four, at least five, or at least six of the Table 1 markers. In some embodiments, MIAMI cells express at least one of CD29, CD81, CD90, or stage-specific embryonic antigen 4 (SSEA4), and at least one of CD122, CD164, hepatocyte growth factor receptor (c-Met), bone morphogenetic protein receptor, type IB (BMP-receptor 1B), neurotrophic tyrosine kinase receptor type 3 (NTRK3), Oct-4, or Rex-1. In other examples, MIAMI cells express at least one of c-Met, BMP-receptor 1B, or NRTK3. In still other examples, MIAMI cells express at least a combination of CD29, CD81, CD90, CD122, and CD164.

The ne-MIAMI cells are generally small cells. In some examples, a ne-MIAMI cell is between about 5 µm and about 12 µm, such as between about 7 µm and about 10 µm ne-MIAMI cells typically contain relatively little cytoplasm and are highly proliferative. In some examples, ne-MIAMI cells have a population doubling time of about 20 hours to about 36 hours.

ne-MIAMI cells can be isolated from a living or postmortem mammal of any age. A mammal includes either human or non-human mammals. In some examples, ne-MIAMI cells are isolated from post-natal subjects, such as a neonate, a child, an adolescent or an adult (including, without limitation, an aged adult) of any mammalian species. In particular embodiments, ne-MIAMI cells are isolated from an adult primate, such as a human.

A subject from whom a biological sample is collected may be a postmortem subject. In particular examples, a postmortem subject is recently deceased. Death of a subject may be determined by any standard known in the art, such as cessation of heart function, or cessation of brain function. In some embodiments, a postmortem subject is one whose heart has recently (such as within about 1 to about 4 hours) stopped beating, or a subject who has no measurable brain activity, or a subject intended for organ or tissue donation. In other examples, a postmortem subject may have been dead for up to 48 hours, such as up to 24 hours or up to 12 hours. In particular examples, a postmortem subject may be housed in a cold environment, such as between about 0 degrees to about 15 degrees C., between about 1 degrees C. to about 10 degrees C., or between about 1 degrees C. to about 5 degrees C., prior to collection of a biological sample.

ne-MIAMI cells may be isolated from one or more biological sample(s), such as bone marrow. Methods for collection of a biological sample will vary depending upon, for example, the type of sample to be collected. Such collection methods are well known in the art. For instance, bone marrow may be collected by inserting a needle into the marrow cavity of a bone under local anesthetic and aspirating marrow from the bone. It may be useful (though not required) to maintain sterile conditions during collection of a sample to reduce the possibility of bacterial, fungal or other infection in a subsequent cell culture of ne-MIAMI cells (see below).

ne-MIAMI cells may be isolated from a biological sample by any method known in the art or a combination thereof, including without limitation the methods disclosed herein (see, for example, Example 1). In one embodiment, ne-MIAMI cells can be selectively expanded using cell culture techniques. In other embodiments, ne-MIAMI cells can be isolated from a biological sample based on the physical properties of the ne-MIAMI cells. For example, several techniques are known in the art by which ne-MIAMI cells may be isolated based on the unique set of markers expressed by ne-MIAMI cells, including, for example, fluorescence-activated cell sorting (FACS), immobilized marker-specific antibodies (such as, antibody-coupled beads), or magnetic-activated cell sorting (MACS).

ne-MIAMI cells may also be isolated on the basis of other physical properties of the ne-MIAMI cells, such as cell size. A biological sample can be sorted on the basis of cell size using any method known in the art. For example, cells in a biological sample may be passed through one or more filters of varying pore size, including filters having a larger pore size, such as of about 50-200 µm, or about 80-100 µm, or filters having a smaller pore size, such as of about 10-50 µm or 20-40 µm. In some examples, sequential filters having decreasing pore size may be employed. In one embodiment, the cells passed through one or more filters are less than 40 µm in diameter. In other embodiments, isolated cells are between about 5 µm and 12 µm in diameter. The cellular component of a biological sample can also be sorted by size by passing a cell population through one or more size-exclusion column(s). In one such embodiment, the cells are eluted along a size gradient such that the largest cells are eluted first and the smallest cells are eluted last. The cells can also be sorted by size using FACS. ne-MIAMI cells may comprise more than about 10%, about 25%, about 50%, about 90% of size-sorted cell sample.

Methods for isolation and expansion of ne-MIAMI cells have been identified and are disclosed herein. A method of isolation of the ne-MIAMI cells includes obtaining a cell population from one or more biological sample(s), such as bone marrow, vertebral bodies, peripheral blood, umbilical cord blood, iliac crest aspirate, fat, cartilage, muscle, skin, bone, teeth, liver or brain Methods for collecting a biological sample useful in the disclosed methods are the same as discussed above.

A biological sample, optionally, may be partially purified after collection. For example, non-cellular materials or dead or damaged cells may be removed by any technique known in the art.

Though not bound by theory, it is believed that ne-MIAMI cells respond favorably to signals (whether humoral, physical, or other signals) produced by one or more other cell types that reside with ne-MIAMI cells within a biological compartment (from which a biological sample is taken); thus, isolation of ne-MIAMI cells may be enhanced by maintaining ne-MIAMI cells and such other resident cell type(s) in proximity to one another and/or in functional contact. In some embodiments, unfractionated cellular components of a biological sample will be retained for subsequent isolation of ne-MIAMI cells. In other embodiments, a biological sample may be fractionated so as to co-fractionate ne-MIAMI cells and any other cell types functionally relevant to ne-MIAMI cells viability. In yet another embodiment, a biological sample may be fractionated to selectively remove cells (or other components) that do not reside with ne-MIAMI cells, but which may be inadvertently included in a sample as a result of a particular cell collection process, such as connective tissue, or mature red blood cells.

In one specific, non-limiting example, the resulting population of cells includes ne-MIAMI cells as greater than 50% of the population, greater than 80% of the population, greater than 90% of the population, or greater than 95% of the population.

The use of adult cells for the treatment of clinical conditions arising from disease, injury, degeneration, and aging has become increasingly effective and sought-after. A remaining key limitation is the identification and isolation of a suitable primary post-natal multilineage-inducible cell population with a homogeneous phenotypic profile and consistently potent regenerative/reparative capacity. The inventors had isolated and patented a novel human cell population termed marrow-isolated adult multilineage-inducible (MIAMI) cells (US Patent No. 7,807,458, October 5, 2010) characterized by a unique molecular profile which distinguish them from other human adult cells. They have demonstrated the effectiveness of MIAMI cells in the treatment of several vascular, neurological, and musculoskeletal conditions using various in vitro and animal models.

It is well established that most cellular therapies require plating and ex-vivo expansion of the cells before transplantation, the regulatory hurdles for these therapies are enormous and can cost several millions of dollars to prove safety and efficacy. Through much scientific work, the present inventors have identified a commercially available antibody which recognizes a marrow-isolated adult multilineage-inducible marker. This antibody is suitable for the isolation of non-expanded marrow-isolated adult multilineage-inducible (ne-MIAMI) cells directly from human whole bone-marrow (BM) samples. Approximately 3-4% of marrow derived total nuclear cells (TNC) appear to express this marker. This finding establishes the conditions for the efficient isolation of sufficient numbers of ne-MIAMI cells from bone marrow (BM) of living or cadaveric donors with minimal manipulation. This allows these ne-MIAMI cells to be considered a human cell and tissue product (HCT/P), as in 361 HCT/P or 351 HCT/P.

In one embodiment, the isolation of ne-MIAMI cells was performed on fresh human BM. After Ficoll gradient separation total nuclear cells (MNCs) were incubated for 30 minutes at 4 degrees C in the presence of anti-SSEA-4 antibody phycoerythrin conjugated (SSEA-4-PE). At the end of the incubation time anti-PE microbeads (Miltenyi Biotech) were added to cells. FACS analysis demonstrated that 3.5% of TNCs are SSEA-4+ cells, as shown in figure 1B, which is about 3,500 times more cells than the number of marrow stromal cells (MSCs) isolated from BM using the empirical isolation procedure used in the inventor's prior patented invention. Thus, the new procedure allows the direct isolation of a greater amount of cells, without the need for expansion. Subsequently, cells were applied to the magnetic separation column of a MiniMACS kit (Miltenyi Biotech). The final eluted product contains the 99.3% of SSEA-4+ cells, containing the ne-MIAMI cells, as shown in figure 1D. After plating, the ne-MIAMI cells acquire the typical morphology similar to the original patented MIAMI cells within 24 hours, as shown in figures 2A and 2B. To verify the differentiation capacity of ne-MIAMI cells, cells were plated in the presence of osteogenic differentiation medium for 21 days. After adherence to plastic surface ne-MIAMI cells start to grow rapidly, as shown in figures 2A and 2B. At the end of the incubation time cells were stained for osteoblastic markers, such alkaline phosphatase and calcium deposition. Osteoblastic-differentiated ne-MIAMI cells express high levels of alkaline phosphatase, as shown in figure 2C; and calcium deposition, as shown in figure 2D. This data confirmed that ne-MIAMI cells are able to differentiate.

In another embodiment the ne-MIAMI cells are isolated from mononuclear cells separated from bone marrow by differential adhesion to substrates. The substrates can be cell culture vessels, bone particles, surfaces coated with extracellular matrix proteins, bone surfaces, etc. Based on the differential attachment properties of ne-MIAMI cells, these can be separated from other cells present in the MNC population by their capacity to adhere to the surface of these substrates with unique affinities. While most leukocytes cells fail to attach or adhere to these surfaces, ne-MIAMI cells attach to these substrates after incubating the MNC population for a period of time, while other cells do not attach effectively. The period of attachment time can range from a few hours (i.e., 4-hrs) to a few days (i.e., 3 to 14 days). After adhesion to the substrates, the non-attached cells are removed by washing with saline solution (i.e., DPBS, HBSS, etc.), while the ne-MIAMI cells remain attached. In addition, to ne-MIAMI cells, other cells (i.e., monocytes, macrophages, osteoclasts, pre-osteoclasts, etc.) can also attach to these surfaces, however, with a different affinity. Ne-MIAMI cells can be separated from these other cell types by exposing the cells to a specific detachment agent. Detachment agents that selectively allow detachment of ne-MIAMI cells, but not the other attached cells, include Ca²⁺ ion chelating agents (such as EDTA, EGTA), a solution composed of concentration low citric acid in combination potassium chloride, or a combination of the above.

The expression of original MIAMI cell markers after plastic adherence and expansion of the ne-MIAMI cells isolated and compared them to MIAMI cells isolated with the original method. Ne-MIAMI cells appear to be a more immature population. The number of cells expressing SSEA-4 was almost double in ne-MIAMI cells compared to adherence-isolated (ad)-MIAMI cells. Other markers, such as CD29, CD105, CD63, CD71, and CD164 were lower in ne-MIAMI cells, this imply that ne-MIAMI cells delay the expression of markers of more committed cells.

The ne-MIAMI cells are anticipated to be capable of preventing tissue damage and promoting tissue repair and functional recovery in animal models of cerebral ischemia, Parkinson's disease, critical limb ischemia, cardio vascular ischemia, vascular disease and coronary artery disease. The ne-MIAMI cells will be similar to the original ad-MIAMI cells in that they secrete a number of cytokines known to be involved in angiogenesis, cell survival, progenitor cell recruitment, immunomodulation and neuroprotection. A fraction of the engrafted cells have expressed features of neuronal or vascular cells.

The purpose of this invention is to provide a unique and novel procedure for isolating marrow-isolated adult multilineage-inducible cells directly from bone marrow without any in vitro expansion. The ne-MIAMI cells have similar, but their own unique characteristics when compared to the expanded MIAMI cells. These inventive ne-MIAMI cells have a broad differentiation potential. The ne-MIAMI cells can be utilized in autologous and allogeneic cellular therapies and tissue engineering strategies tailored to the needs of an individual, independently of his/her age, aimed at repairing damaged or diseased organs and tissues.

Most cellular therapies require plating and ex-vivo expansion of the cells before transplantation, the regulatory hurdles for these therapies are enormous and can cost several millions of dollars to prove safety and efficacy. There are several companies offering culture-expanded adult multilineage cells. These companies could benefit by using the ne-MIAMI cell product as an unexpanded source of multilineage cells, which can be modified by their own proprietary culturing and expansion procedures which could provide expression markers on the more primitive cells of the ne-MIAMI invention further refining the cell differentiation.

The ne-MIAMI cells are derived from an easily accessible source, such as bone marrow. The ne-MIAMI cells when expanded have similar, but unique characteristics compared to the expanded MIAMI cells. They have a broad differentiation potential. The ne-MIAMI cells can be utilized in autologous and allogeneic cellular therapies and tissue engineering strategies tailored to the needs of an individual, independently of his/her age, aimed at repairing damaged or diseased organs and tissues. The ne-MIAMI cells like the expanded MIAMI cells express markers for all three embryonic germ layers; they could be differentiated towards all different cell types derived from all three germ-layers found in the body. Ne-MIAMI cells could also be used to repopulate cell types lost to chemo or radiation therapy after treatment of cancers or malignancies, Ne-MIAMI cells could also be used in co-transplantation approaches with other organ/tissue during allo-transplantation procedure to reduce graft versus host disease (GvHD). A comparison of markers expressed after expansion by ne-MIAMI cells and ad-MIAMI cells is shown in Table 2 below.

**Table 2: Markers expressed by ne-MIAMI cells after expansion**

| | Marker | ne-MIAMI cells | ad-MIAMI cells |
|---|---|---|---|
| 1 | SSEA-4 | 56.1% | 32.1% |
| 2 | CD29 | 86% | 93.5% |
| 3 | CD73 | 99.3% | 99.5% |
| 4 | CD90 | 98.7% | 99.2% |
| 5 | CD105 | 25% | 34.8% |
| 6 | CD63 | 5.3% | 35.3% |
| 7 | CD71 | 3.4% | 9.4% |
| 8 | CD81 | 99.7% | 99.8% |
| 9 | CD164 | 9.4% | 20% |
| 10 | CD34 | Negative | Negative |
| 11 | CD45 | Negative | Negative |
| 12 | MHC-1 | Negative | Negative |
| 13 | HLA-DR | Negative | Negative |

It is estimated that multilineage cell therapy will help approximately 130 million people in the United States.

It is believed the ne-MIAMI cells could have an impact in biomedicine as broad as pluripotent multilineage cells.

Variations in the present invention are possible in light of the description of it provided herein. While certain representative embodiments and details have been shown for the purpose of illustrating the subject invention, it will be apparent to those skilled in this art that various changes and modifications can be made therein without departing from the scope of the subject invention. It is, therefore, to be understood that changes can be made in the particular embodiments described, which will be within the full intended scope of the invention as defined by the following appended claims.

## Claims

1. A process for isolating non-expanded post-natal multilineage-inducible cells comprising:
isolating bone marrow from an isolated biological sample;
isolating total nuclear cells (TNCs) obtained from said bone marrow;
incubating the TNCs in the presence of an antibody directed to a cell surface marker selective for non-expanded post-natal multilineage-inducible cells;
separating said cells to isolate non-expanded post-natal multilineage-inducible cells; and
isolating said non-expanded post-natal multilineage-inducible cells in the absence of expansion,
**characterized in that** said antibody is directed to SSEA-4 and that said cell surface marker comprises SSEA-4.

2. Process according to claim 1, wherein said step of separating cells to isolate non-expanded post-natal multilineage-inducible cells comprises separation of said cells by magnetic or cell sorting means.

3. Process according to claim 1, wherein said step of separating cells to isolate non-expanded post-natal multilineage-inducible cells comprises separation by differential cell adhesion using selective detachment solutions.

4. Process according to claim 3, wherein said detachment solutions are selected from enzymes, citric acid, mild acids or divalent ion chelating agents or combinations thereof.

5. Process according to claim 4, wherein said detachment solution is selected as enzymes, said enzymes being selected from trypsin, collagenases or other mild proteases.

6. Process according to claim 4, wherein said detachment solution is a mild acid, said mild acid being selected from citric acid, potassium chloride or combinations thereof.

7. Process according to claim 4, wherein said detachment solution is a chelating agent, said chelating agent being selected from EDTA, EGTA or combinations thereof.

8. Process according to claim 1, wherein said non-expanded post-natal multilineage-inducible cells collected yield 1.5% or greater of the total nuclear cells.

9. Process according to claim 1, wherein said non-expanded post-natal multilineage-inducible cells exhibit characteristics when expanded after being isolated, by expressing phenotype markers SSEA-4, CD29, CD105, CD63, CD71 and CD164 post expansion.

10. Process according to claim 1, wherein said non-expanded post-natal multilineage-inducible cells are multipotent cell precursors.

11. Process according to claim 1, wherein the animal is a human donor.

## Patentansprüche

1. Verfahren zum Isolieren von nicht-expandierten postnatalen multlineage-induzierbaren Zellen, umfassend:
Isolieren von Knochenmark aus einer isolierten biologischen Probe;
Isolierung von aus dem Knochenmark erhaltenen Gesamtkernzellen (TNCs);
Inkubieren der TNCs in Gegenwart eines Antikörpers, der gegen einen Zelloberflächenmarker gerichtet ist, der für nicht-expandierte postnatale multlineage-induzierbare Zellen selektiv ist;
Abtrennen der Zellen, um nicht-expandierte postnatale multlineage-induzierbare Zellen zu isolieren; und
Isolieren der nicht-expandierten postnatalen multlineage-induzierbaren Zellen unter der Abwesenheit von Expansion,
**dadurch gekennzeichnet, dass** der Antikörper gegen SSEA-4 gerichtet ist und dass der Zelloberflächenmarker SSEA-4 umfasst.

2. Verfahren nach Anspruch 1, wobei der Schritt des Abtrennens der nicht-expandierten postnatalen multlineage-induzierbaren Zellen das Abtrennen der Zellen durch magnetische oder Zellsortiermittel umfasst.

3. Verfahren nach Anspruch 1, wobei der Schritt des Abtrennens der nicht-expandierten postnatalen multlineage-induzierbaren Zellen das Abtrennen durch differentielle Zelladhäsion unter Verwendung von selektiven Ablöselösungen umfasst.

4. Verfahren nach Anspruch 3, wobei die Ablöselösungen aus Enzymen, Zitronensäure, milden Säuren oder zweiwertigen lonenchelatbildnern oder Kombinationen davon ausgewählt sind.

5. Verfahren nach Anspruch 4, wobei die Ablöselösung aus Enzymen ausgewählt ist, wobei die Enzyme aus Trypsin, Kollagenasen oder anderen milden Proteasen ausgewählt sind.

6. Verfahren nach Anspruch 4, wobei die Ablöselösung eine milde Säure ist, wobei die milde Säure aus Zitronensäure, Kaliumchlorid oder Kombinationen davon ausgewählt ist.

7. Verfahren nach Anspruch 4, wobei die Ablöselösung ein Chelatbildner ist, wobei der Chelatbildner aus EDTA, EGTA oder Kombinationen davon ausgewählt ist.

8. Verfahren nach Anspruch 1, wobei die gesammelten nicht-expandierten postnatalen multlineage-induzierbaren Zellen 1,5% oder mehr der Gesamtkernzellen ergeben.

9. Verfahren nach Anspruch 1, wobei die nicht-expandierten postnatalen multlineage-induzierbaren Zellen Eigenschaften aufweisen, wenn sie nach Isolation expandiert werden, durch Expression der Phänotyp-Marker SSEA-4, CD29, CD105, CD63, CD71 und CD164 nach Expansion.

10. Verfahren nach Anspruch 1, wobei die nicht-expandierten postnatalen multlineage-induzierbaren Zellen multipotente Zellvorläufer sind.

11. Verfahren nach Anspruch 1, wobei das Tier ein menschlicher Spender ist.

## Revendications

1. Procédé d'isolement de cellules post-natales non amplifiées inductibles par multilignées comprenant les étapes consistant à :
isoler de la moelle osseuse à partir d'un échantillon biologique isolé ;
isoler des cellules nucléaires totales (TNC) obtenues à partir de ladite moelle osseuse ;
incuber les TNC en présence d'un anticorps dirigé contre un marqueur de surface cellulaire sélectif pour des cellules post-natales non amplifiées inductibles par multilignées ;
séparer lesdites cellules pour isoler des cellules post-natales non amplifiées inductibles par multilignées ; et
isoler lesdites cellules post-natales non amplifiées inductibles par multilignées en l'absence d'amplification,
**caractérisé en ce que** ledit anticorps est dirigé contre SSEA-4 et **en ce que** ledit marqueur de surface cellulaire comprend SSEA-4.

2. Procédé selon la revendication 1, dans lequel ladite étape de séparation de cellules pour isoler des cellules post-natales non amplifiées inductibles par multilignées comprend la séparation desdites cellules par des moyens de tri magnétique ou cellulaire.

3. Procédé selon la revendication 1, dans lequel ladite étape de séparation de cellules pour isoler des cellules post-natales non amplifiées inductibles par multilignées comprend la séparation par adhésion cellulaire différentielle en utilisant des solutions de dissociation sélective.

4. Procédé selon la revendication 3, dans lequel lesdites solutions de dissociation sont choisies parmi des enzymes, de l'acide citrique, des acides doux ou des agents chélateurs d'ions divalents ou des combinaisons de ceux-ci.

5. Procédé selon la revendication 4, dans lequel ladite solution de dissociation est choisie sous forme d'enzymes, lesdites enzymes étant choisies parmi de la trypsine, des collagénases ou d'autres protéases légères.

6. Procédé selon la revendication 4, dans lequel ladite solution de dissociation est un acide doux, ledit acide doux étant choisi parmi de l'acide citrique, du chlorure de potassium ou des combinaisons de ceux-ci.

7. Procédé selon la revendication 4, dans lequel ladite solution de dissociation est un agent chélateur, ledit agent chélateur étant choisi parmi EDTA, EGTA ou des combinaisons de ceux-ci.

8. Procédé selon la revendication 1, dans lequel lesdites cellules post-natales non amplifiées inductibles par multilignées collectées génèrent 1,5 % ou plus des cellules nucléaires totales.

9. Procédé selon la revendication 1, dans lequel lesdites cellules post-natales non amplifiées inductibles par multilignées présentent des caractéristiques lorsqu'elles sont amplifiées après avoir été isolées, en exprimant des marqueurs phénotypiques SSEA-4, CD29, CD105, CD63, CD71 et CD164 après amplification.

10. Procédé selon la revendication 1, dans lequel lesdites cellules post-natales non amplifiées inductibles par multilignées sont des précurseurs de cellules multipotentes.

11. Procédé selon la revendication 1, dans lequel l'animal est un donneur humain.
